# EUROPEAN PATENT APPLICATION

(11) **EP 2 208 456 A1**
(43) Date of publication of application: **21.07.2010**
(21) Application number: 10151075.8
(22) Date of filing: 19.01.2010
(51) Int. Cl.: A61B 1/05, H05K 1/18

(54) **Endoscopic device**

(30) Priority: 19.01.2009 TW 98101913
(71) Applicant: Chang, Hui-Yu, Taipei (TW)
(72) Inventor: Chang, Hui-Yu, Taipei (TW)
(74) Representative: Baldwin, Mark

(57) **Abstract**

An endoscopic device includes a flexible PCB (10) adapted to fold to form a stacked structure with a plurality of spaced layers. The PCB (10) comprises a plurality of units, at least one interconnection (111) interconnecting any two adjacent units, and a plurality of connections connecting to a conductor (53, 63). Advantageously, the connections can reduce space for mounting electronic devices on the units. The connections each comprise a first section extending from the connected unit, and a second section extending from the first section with a passive electronic component (52, 62) being disposed thereon. Further, the conductors (53, 63) extend from the second sections of the connections.

## Description

### BACKGROUND OF THE INVENTION

### 1. Field of Invention

The invention relates to endoscopic devices and more particularly to such an endoscopic device with improved characteristics.

### 2. Description of Related Art

Endoscopic tools for conducting medical examination within the human body cavities are well known in the art. For example, U.S. Publication Application No. 2006/0189844 discloses an endoscopic device. Drawbacks thereof are described below. Areas for soldering on a PCB (printed circuit board) are required. This makes a reduction of PCB size impossible. Further, a length about 30mm is formed by folding buses. Such length can adversely limit a moving range of the endoscopic device within the body organs. For example, a doctor cannot observe the larynx and trachea by means of the endoscopic device.

The above drawbacks are common among typical endoscopic devices and are not solved. Thus, continuing improvements in the exploitation of endoscopic device are constantly being sought.

### SUMMARY OF THE INVENTION

Embodiments of the invention may include an endoscopic device comprising an image capturing unit and a flexible circuit board that comprises at least two circuit board units and at least one flexible connection between said circuit board units, said flexible circuit board unit being foldable between a non-folded pre-assembly condition and a folded condition assembly condition in which it is assembled in the endoscopic device and in which said circuit board units form a layered structure. Preferably when in said folded condition said circuit board units are disposed one above the other. Preferably when in said unfolded condition said flexible circuit board may be in a planar configuration. Preferably, at least one of the circuit board units is provided with a flexible extension member that may be connected with an external conductor. The flexible extension member may carry one or more electronic components, typically passive electronic components, By having components mounted on such a flexible extension member, which extends in the lengthways direction of the endoscopic device when the device is assembled, it is possible to reduce the diameter of the layered circuit board structure and thereby the overall diameter of the endoscopic device.

It is therefore one object of the invention to provide an endoscopic device that can have a lens with a diameter less than 5mm. The endoscopic device may be cost effective so that it can be discarded after use so as to prevent cross-infection from occurring.

Preferably, the endoscopic device has a diameter less than 4.3mm.

It is another object of the invention to provide an endoscopic device that can have a lens with length less than 10mm. Lens length is an important factor for determining quality of the endoscopic device. In general, a shorter length makes the endoscopic device easier to be inserted into narrow body cavities for medical examination. The shortest lens length may be about 10mm in the invention.

Preferably, the endoscopic device comprises a flexible PCB adapted to fold to form a stacked structure with a plurality of spaced layers. The PCB comprises a plurality of units, at least one interconnection interconnecting any two adjacent units, and a plurality of connections connecting to a conductor. Advantageously, the connections can reduce space for mounting electronic devices on the units. The connections each comprise a first section extending from the connected unit, and a second section extending from the first section with a passive electronic component being disposed thereon. Further, a conductor extends from the second section of the connection.

Preferably, when bent the interconnections have a length about the same as the height of two adjacent units. This enables the PCB to fold as a stacked structure with a plurality of spaced layers. That is, it is an unbending section. The connections are adapted to extend downward from the stacked structure. The first section of the connection has a length greater than 3mm of the height of the unbending section.

Preferably, larger active electronic components such as microcontrollers and sensors are mounted on the units. Smaller passive electronic components such as resistors and capacitors can be mounted in the second sections of the connections. This can eliminate drawbacks of the prior art endoscopic devices which comprise active and passive electronic components mounted on the PCB making a great reduction of PCB area and lens diameter impossible.

Preferably, the first sections of the connections have a length greater than the height of the unbending section so that electronic components mounted on the first sections of the connections are disposed externally of the stacked units. A conductor is extended from the second section of each connection for signal transmission. Each of the interconnections may have a width of about 2mm. Each of the connections may have a width about 2mm. Each of the interconnections and the connections extends from the periphery of the unit. Thus, after folding, the interconnections and the connections do not increase the size of the lens. This can be a useful characteristic of embodiments of the invention.

Embodiments of the invention can have the following advantageous features:

The units of the flexible PCB have larger active electronic components such as microcontrollers and sensors mounted thereon. Other electronic components, typically passive components, can be mounted on the flexible connections that extend from the PCB units making it possible to reduce the PCB size to a minimum and so reduce the overall diameter of the endoscope device.

The active electronic components mounted on the units can be in signal communication with external devices via the conductors extending out of the second sections of the connection. Hence, a reduction of conductor length is made possible. Further, the layout area can be distributed, resulting in a size reduction of the PCB including the units.

The bending angle of each of the interconnections and the connections can be a minimum so as to greatly decrease the PCB size.

The units with active electronic components mounted thereon can be folded to form a stacked structure because the interconnections have a length sufficient to allow folding. This has the effect of greatly decreasing the height of the non-bending section and increasing the operating space of the endoscopic device such that it can be capable of examining the larynx and trachea.

The connections and conductors (e.g., small wires) extending therefrom are flexible in nature. This means that except for the non-bending section they are bendable. Further, their bending angles are much smaller than that of the conventional fibre optic camera.

The above and other objects, features and advantages of embodiments of the invention will become apparent from the following detailed description and drawings, which are given by way of example only.

### BRIEF DESCRIPTION OF THE DRAWINGS

FIG. 1 is an exploded view of a first preferred embodiment of an endoscopic device according to the invention;
FIG. 2 is a perspective view of the assembled endoscopic device of FIG. 1;
FIG. 3 is a top plan view of the fully extended PCB of FIG. 1;
FIG. 3A is a side elevation of the first and the second unit of FIG. 3 upon being folded;
FIG. 4 is a top plan view of the fully extended PCB of a second preferred embodiment of an endoscopic device;
FIG. 4A is a side elevation of the PCB of FIG. 4 in a folded condition;
FIG. 5 is a perspective view of the PCB of the second preferred embodiment;
FIG. 6 is a top plan view showing a modification of the PCB of FIG.4;
FIG. 6A is a side elevation of the PCB of FIG. 6 in a folded condition;
FIG. 7 is a top plan view of the fully extended PCB of a third preferred embodiment of an endoscopic device;
FIG. 7A is a side elevation of the PCB of FIG. 7 in a folded condition;
FIG. 8 is an exploded view of the third preferred embodiment of endoscopic device;
FIG. 9 is a perspective view of the assembled endoscopic device of FIG. 8;
FIG. 10 is a top plan view showing a modification of the PCB of FIG. 7; and
FIG. 10A is a side elevation of the PCB of FIG. 10 in a folded condition.

### DETAILED DESCRIPTION OF THE INVENTION

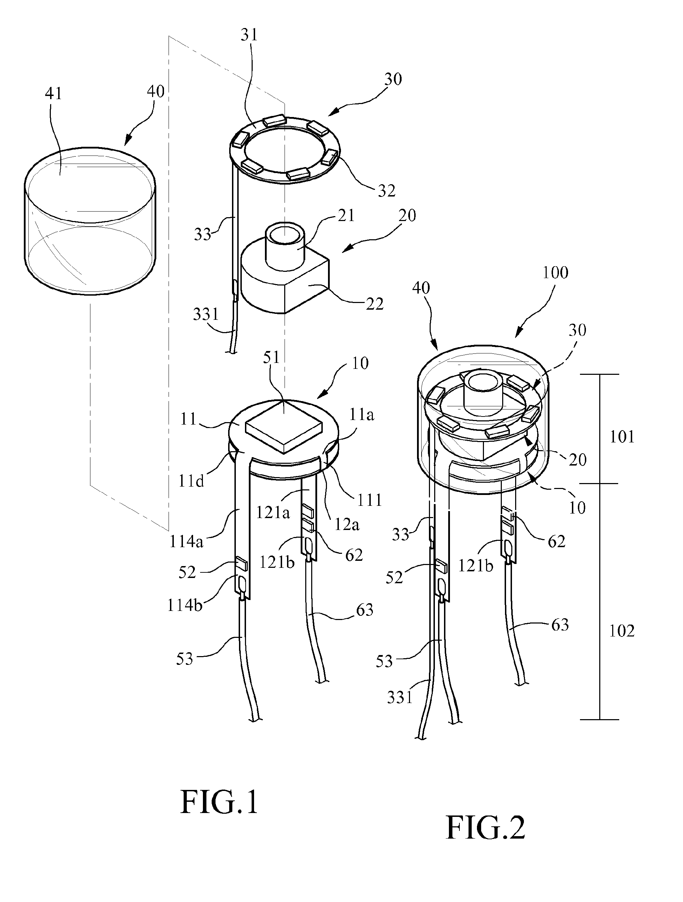

Referring to FIGS. 1, 2, 3, and 3A, an endoscopic device 100 in accordance with a first preferred embodiment of the invention comprises the following components as discussed in detail below.

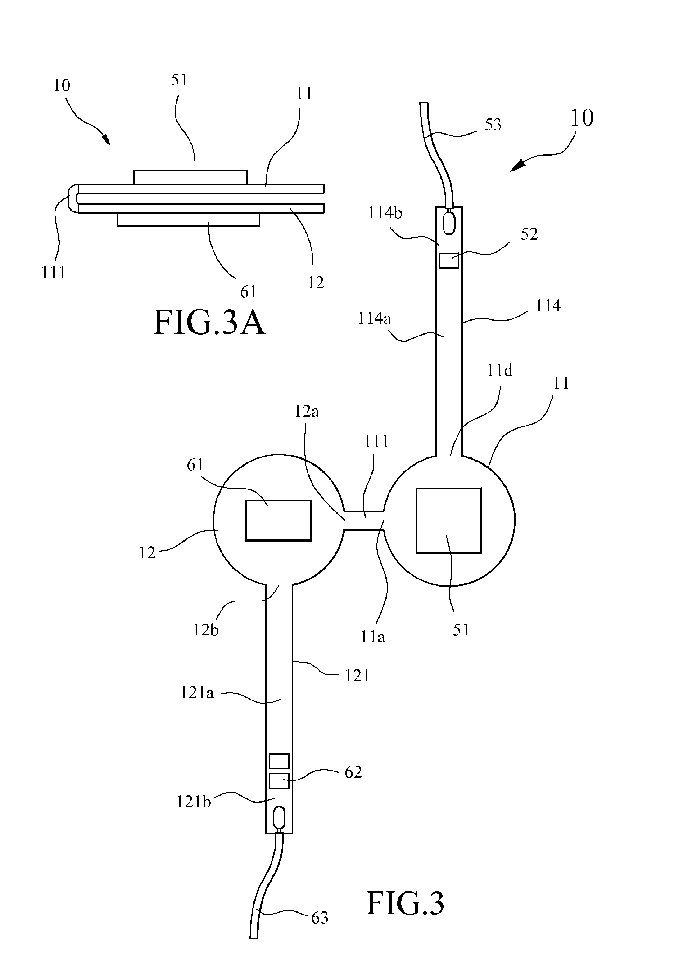

A flexible PCB 10 can be folded to form a two spaced-layer stack (see FIG. 3A). An image capturing assembly 20 for image capture and processing comprises a sector-shaped seat 22 and a cylindrical lens 21 provided on the seat 22. A light source 30 comprises a ring-shaped electronic member 31, a plurality of spaced light emitting elements (e.g., LEDs (light-emitting diodes) or OLED (organic light-emitting diodes)) 32 provided on the electronic member 31, and a wire 33 extending downward out of the bottom of the electronic member 31. A conductor 331 is connected to one end of the wire 33. The seat 22 is seated on the PCB 10. The electronic member 31 is rests upon the seat 22 with the lens 21 passing through the electronic member.

An inverted cup-shaped cover 40 covers the assembled PCB 10, the image capturing assembly 20, and the light source 30. The cover 40 has a transparent member 41 on top so that light emitted by the light emitting elements 32 can pass through the transparent member 41 and external light passing through the transparent member 41 can be captured by the lens 21. Preferably, the cover 40 has a diameter less than 5mm. More preferably, the cover 40 has a diameter less than 4.3mm.

As shown in FIG. 2, the assembled endoscopic device 100 is composed of a relatively rigid non-bending section 101 and a bendable section 102. Portions of the PCB 10, the image capturing assembly 20, and the light source 30 in the cover 40 are disposed in the non-bending section 101. Preferably, the non-bending section 101 has a length less than 10mm.

As shown in FIG. 3, the PCB 10 comprises a first unit 11 and a second unit 12 that are interconnected. Note that each unit is shaped as a disc.

The first unit 11 comprises a bridge point 11a, and a connecting point 11d spaced around a periphery thereof, the second unit 12 comprises a bridge point 12a and a connecting point 12b spaced from the bridge point 12a about 90 degrees on a periphery thereof. A first interconnection 111 connects the bridge points 11a and 12a, and has a width of at most 2mm.

As shown in FIGS. 1 and 3A, a first active electronic component 51 is provided on the top of the first unit 11 and a second active electronic component 61 is provided on the underside of the second unit 12. The first active electronic component 51 is received within the seat 22. The second unit 12 is provided under the first unit 11 by bending the first interconnection 111.

Alternatively, the second active electronic component 61 can be provided on top of the second unit 12 (not shown).

In FIG. 3, the first connection 114 extends from the connecting point 11 d and a second connection 121 extends from the connecting point 12b.

The first connection 114 comprises a first section 114a extending from the connecting point 11d, and a second section 114b extending from the first section 114a. A first passive electronic component 52 is provided on the second section 114b.

The second connection 121 comprises a first section 121a extending from the connecting point 12b, and a second section 121 b extending from the first section 121 a. Two second passive electronic components 62 are provided on the second section 121 b.

In the first embodiment, the width of the first and second connections 114, 121 allows the reduction of the size of the PCB 10 since each connection is adapted to bend smoothly from the periphery of the unit without an unnecessary arcuate portion (as shown in FIG. 1). The second sections 114b, 121 b allow a further reduction of the size of the endoscopic device 100 since the passive electronic components 52, 62 are removed from the first and second units 11, 12.

The first and second connections 114, 121 together with the wire 33 of the light source 30 form the bendable section 102 of the endoscopic device 100 (see FIG. 2).

A conductor 53 extends from the second section 114b and a conductor 63 extends from the second section 121b. Preferably, the first and second sections 121 a, 114a both have a length greater than a distance between the first unit 11 and the second unit 12 to keep the passive electronic components 52, 62 out of the unbending section 101 as show in FIG. 2.

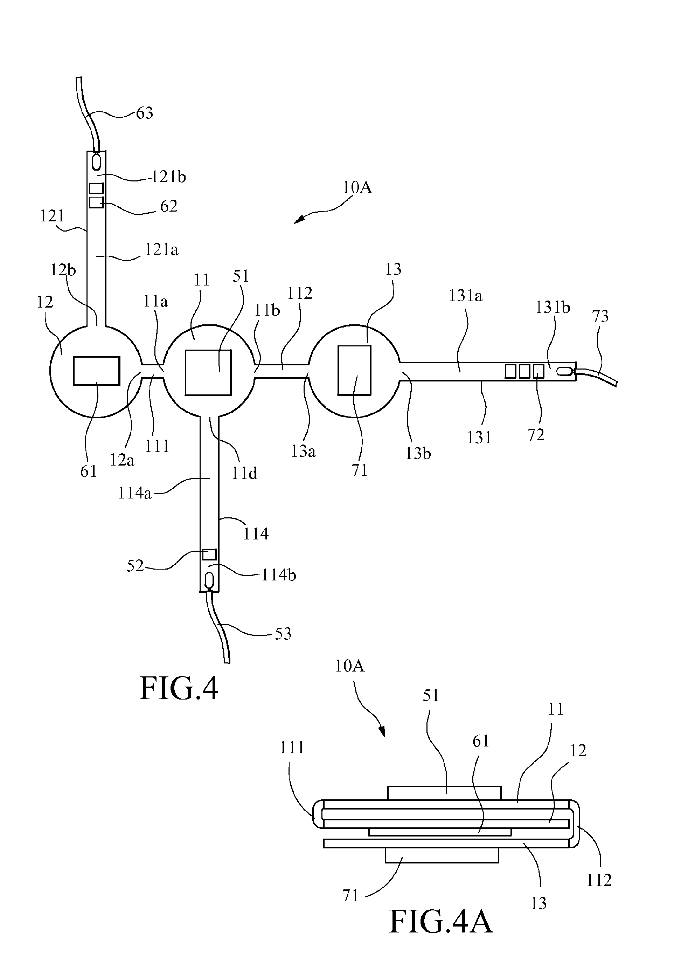

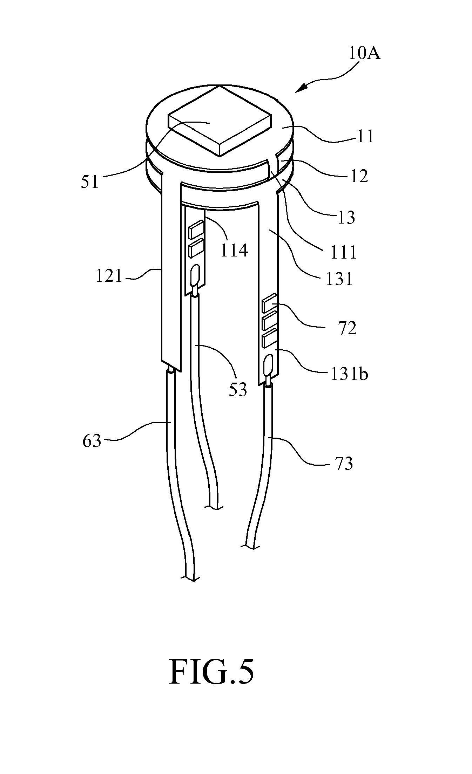

Referring to FIGS. 4, 4A, and 5, a PCB of the endoscopic device 100 in accordance with a second preferred embodiment of the invention comprises the following components as discussed in detail below.

As shown in FIGS. 4 and 4A, the PCB 10A not only comprises a first unit 11, and a second unit 12 in the same way as the first preferred embodiment, but also a third unit 13. The PCB 10A can be folded to form a three-spaced layer stack. Note that each unit is shaped as a disc.

The third unit 13 comprises a bridge point 13a and a connecting point 13b opposing the bridge point 13a on a periphery thereof, a second interconnection 112 interconnecting the bridge point 13a and a bridge point 11 b of the first unit 11, and a third connection 131 extending from the connecting point 13b.

As shown in FIG. 4A, a third active electronic component 71 is provided on the underside side of the third unit 13, which is provided under the second active electronic component 61 by bending the second interconnection 112. This is a stacked structure with three spaced-layers as shown in FIG. 5. Alternatively, the third active electronic component 71 can be provided on top of the third unit 13.

As shown in FIG. 4, the third connection 131 comprises a first section 131 a extending from the connecting point 13b, and a second section 131 b extending from the first section 131 a. Preferably, the first section 131 a has a length greater than the distance between the first unit 11 and the third unit 13 to keep the passive electronic components 72 out of the non-bending section 101.

A third passive electronic component 72 is provided on the second section 131 b, and a conductor 73 extends from the second section 131 b.

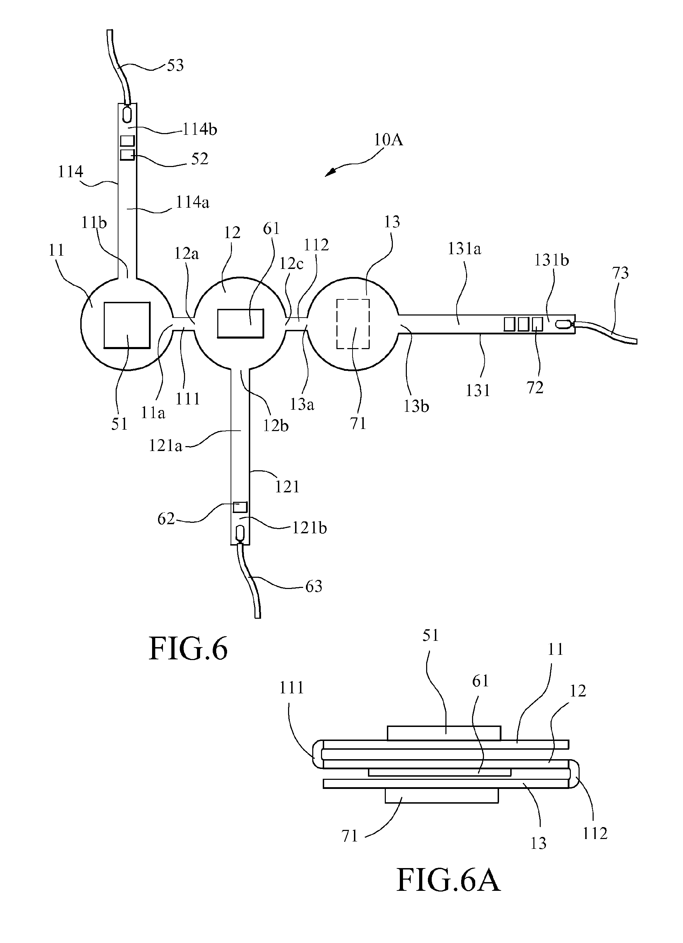

As shown in FIG. 4A, when the PCB 10A is in a folded condition, the first, second and third units 11, 12 and 13 are disposed one above another with the first unit connected to the second unit by the interconnection 111 and the first unit connected to the third unit by the interconnection 112. There is no direct connection between the second unit 12 and the third unit 13. FIGS. 6 and 6A show a modification to the PCB of the second embodiment. The modification is in the arrangement of the interconnections 111 and 112 between the units 11, 12 and 13 and the relative positions of the units when the PCB is in the extended condition shown in FIG. 6. In this case, the first unit 11 is on the left instead of the middle and is followed in series by the second and third units 12, 13 with the first interconnection 111 connecting the first unit to the second unit and the second interconnection 112 connecting the second unit to the third unit. Also, when the PCB is in the extended connection, the component 71 faces in the opposite direction to the components 51, 61. As shown in FIG 6A, when the modified PCB 10A is folded, the first, second and third units 11, 12 and 13 and respective components 51, 61 and 71 are configured in the same way as in FIG. 4A, However, in this case, the first interconnection 111 connects the first unit to the second unit and the second interconnection 112 connects the second unit to the third unit and there is no direct connection between the first unit and the third unit.

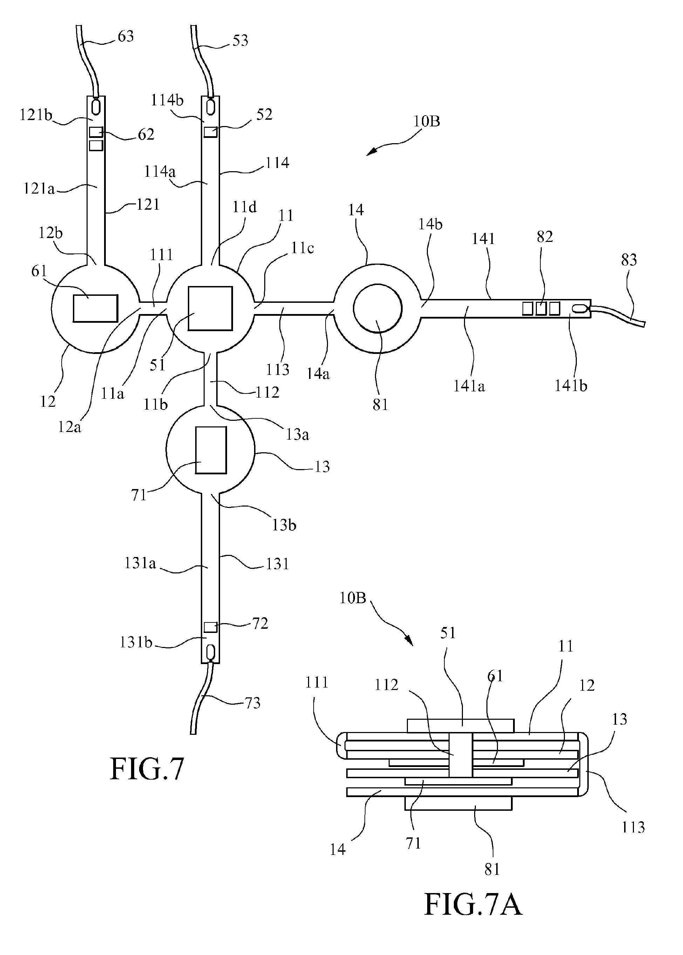

Referring to FIGS. 7, 7A, 8 and 9, an endoscopic device 100 and PCB thereof in accordance with a third preferred embodiment of the invention comprises the following components as discussed in detail below.

As shown in FIGS. 7 and 7A, the PCB 10B not only comprises a first unit 11, a second unit 12 and a third unit 13 in the same way as the second preferred embodiment, but also a fourth unit 14. The PCB 10B can be folded to form a four spaced-layer stack. Note that each unit is shaped as a disc.

The fourth unit 14 comprises a bridge point 14a and a connecting point 14b opposing the bridge point 14a on a periphery thereof, a third interconnection 113 interconnecting the bridge point 14a and a bridge point 11c of the first unit 11 and a fourth connection 141 extending from the connecting point 14b.

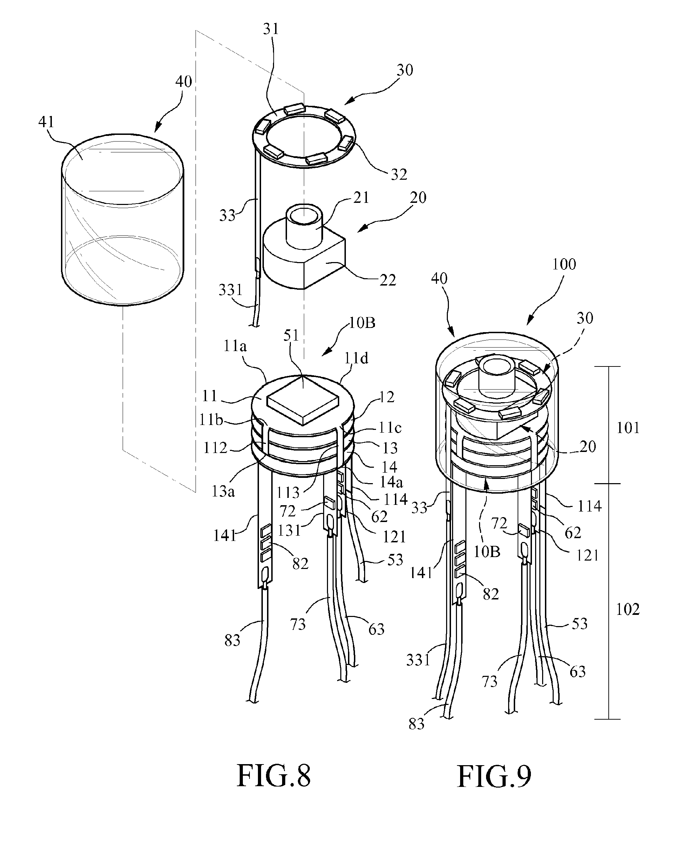

A fourth active electronic component 81 is provided on the underside of the fourth unit 14 which is provided under the third active electronic component 71 by bending the third interconnection 113. This is a stacked structure with four spaced-layers as shown in FIG. 8. Alternatively, the fourth active electronic component 81 can be provided on top of the fourth unit 14.

As shown in FIG. 7, the fourth connection 141 comprises a first section 141 a extending from the connecting point 14b, and a second section 141 b extending from the first section 141 a. Preferably, the first section 141 a has a length greater than the distance between the first unit 11 and the fourth unit 14 to keep the passive electronic components 82 out of the non-bending section 101 as shown in FIG. 9.

A fourth passive electronic component 82 is provided on the second section 141 b, and a conductor 83 extends from the second section 141 b.

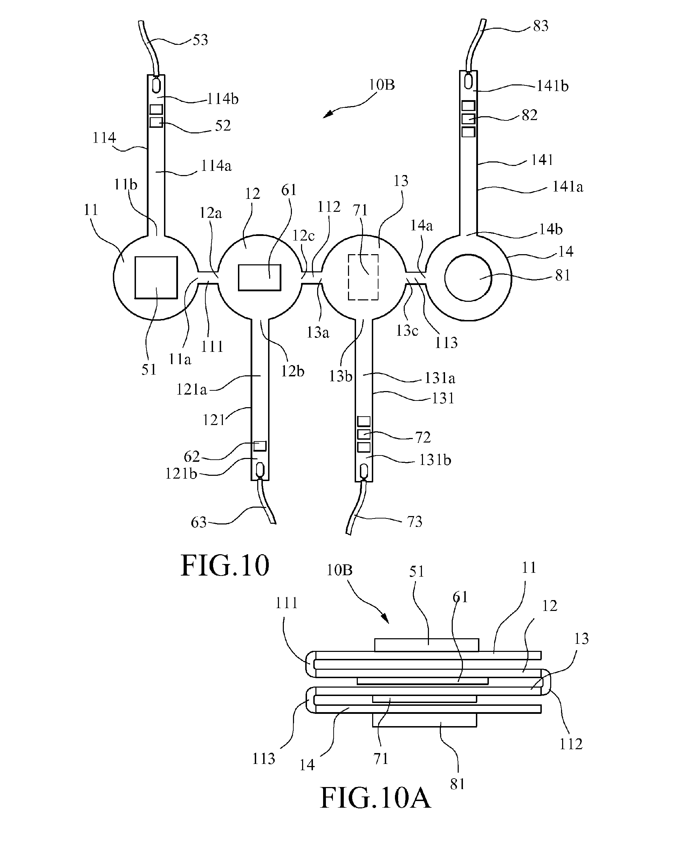

As shown in FIG. 7A, when the PCB 10B is in a folded condition, the first, second, third and fourth units 11, 12, 13 and 14 are disposed one above another with the first unit connected to the second unit by the interconnection 111, the first unit connected to the third unit by the interconnection 112, the first unit connected to the fourth unit by the interconnection 113. There is no direct connection between the second, third and fourth units, which each connect to the first unit only. FIGS. 10 and 10A show a modification to the PCB of the third embodiment. The modification is in the arrangement of the interconnections 111, 112 and 113 between the units 11, 12, 13 and 14 and the relative positions of the units when the PCB is in the extended condition shown in FIG. 6. In this case, the first unit 11 is on the left instead of the middle and is followed in series by the second, third and fourth units 12, 13 and 14 with the first interconnection 111 connecting the first unit to the second unit, the second interconnection 112 connecting the second unit to the third unit and the third interconnection connecting the third unit to the fourth unit. As shown in FIG 10A, when the modified PCB 10B is folded, the first second, third and fourth units 11, 12, 13 and 14 and respective components 51, 61,71 and 81 are configured in the same way as in FIG. 7A. However, in this case, the first interconnection 111 connects the first unit to the second unit, the second interconnection 112 connects the second unit to the third unit and the third interconnection 113 connects the third unit to the fourth unit.

In the above embodiments, each of the units has a diameter of 3.7mm, and each of the connections has a width of at most 2mm. The width of the connections allows the size of the PCB to be reduced since each connection is adapted to bend smoothly from the periphery of the unit without an unnecessary arcuate portion.

While two units, three units and four units are discussed above in the first, second and third embodiments respectively, if micro-electronic technology is further upgraded, a fourth preferred embodiment of the endoscopic device of the present invention having only one unit may be able to provide the same functionality and advantages as the multi-layer structures mentioned above.

While the invention herein disclosed has been described by means of specific embodiments, numerous modifications and variations could be made thereto by those skilled in the art without departing from the scope of the invention set forth in the claims.

## Claims

1. An endoscopic device (100) comprising:
an image capturing assembly (20) comprising a seat (22) and a cylindrical lens (21) disposed on the seat (22);
a light source (30) disposed on the seat (22) for light emission;
a flexible PCB (printed circuit board) (10) comprising:
a first unit (11) comprising a first active electronic component (51) with the image capturing assembly (20) disposed thereon for image capture and processing, the first unit (11) further comprising a first bridge point (11a) and a connecting point (11d) spaced around a periphery thereof;
a second unit (12) having a first side and an opposing second side, the second unit(12) comprising a first bridge point (12a) and a connecting point (12b), both being on a periphery thereof;
a first interconnection (111) interconnecting the first bridge point (11a) of the first unit (11) and the bridge point (12a) of the second unit (12);
a first connection (114) comprising a first section (114a) extending from the connecting point (11d) of the first unit (11), and a second section (114b) extending from the first section (114a) with a first passive electronic component (52) being disposed thereon; and
a second connection (121) comprising a first section (121 a) extending from the connecting point (12b) of the second unit (12), and a second section (121b) extending from the first section (121 a) with a second passive electronic component (62) disposed thereon;
wherein the second unit (12) is disposed under the first unit (11) by bending the first interconnection (111) so as to shape the PCB (10) as a two spaced-layer structure.

2. The endoscopic device (100) of claim 1, wherein the first sections (121 a, 114a) each have a length greater than a distance between the first unit (11) and the second unit (12).

3. The endoscopic device (100) of claim 1 or 2, wherein the first interconnections (111), the first connection (114), and the second connection (121) all have a width of about 2 mm.

4. The endoscopic device (100) of claim 1, 2 or 3, further comprising a second active electronic component (61) disposed on the second unit (12).

5. The endoscopic device (100) of claim 1, further comprising a third unit (13) having a first side and an opposing second side, the third unit (13) comprising a first bridge point (13a) and a connecting point (13b), both being on a periphery thereof, a second interconnection (112) interconnecting a second bridge point (11 b) of the first unit (11) and the bridge point (13a) of the third unit (13), a third connection (131) comprising a first section (131 a) extending from the connecting point (13b) of the third unit (13), and a second section (131b) extending from the first section (131a) with a third passive electronic component (72) disposed thereon, wherein the third unit (13) is disposed under the second side of the second unit (12) by bending the second interconnection (112) so as to shape the PCB as a three spaced-layer structure.

6. The endoscopic device (100) of claim 1, further comprising a third unit (13) having a first side and an opposing second side, and the third unit(13) comprising a first bridge point (13a) and a connecting point (13b), both being on a periphery thereof, a second interconnection (112) interconnecting a second bridge point (12c) of the second unit (12) and the bridge point (13a) of the third unit (13), a third connection (131) comprising a first section (131 a) extending from the connecting point (13b) of the third unit (13), and a second section (131 b) extending from the first section (131 a) with a third passive electronic component (72) disposed thereon,
wherein the third unit (13) is disposed under the second side of the second unit (12) by bending the second interconnection (112) so as to shape the PCB as a three spaced-layer structure.

7. The endoscopic device (100) of claim 5 or 6, wherein the connection (131) has a width about 2mm, and the first section (131 a) has a length greater than a distance between the first unit (11) and the third unit (13).

8. The endoscopic device (100) of claim 5, 6, or 7, further comprising a third active electronic component (71) disposed on the third unit (13).

9. The endoscopic device (100) of any one of claims 5 to 8, further comprising a fourth unit (14) comprising a bridge point (14a) and a connecting point (14b), both being on a periphery thereof, a third interconnection (113) interconnecting a third bridge point (11c) of the first unit (11) and the bridge point (14a) of the fourth unit (14), a fourth connection (141) comprising a first section (141 a) extending from the connecting point (14b) of the fourth unit (14), and a second section (141 b) extending from the first section (141 a) with a fourth passive electronic component (82) disposed thereon, wherein the fourth unit (14) is disposed under the second side of the third unit (13) by bending the fourth interconnection (113) so as to shape the PCB as a four spaced-layer structure.

10. The endoscopic device (100) of any one of claims 5 to 8, further comprising a fourth unit (14) comprising a bridge point (14a) and a connecting point (14b), both being on a periphery thereof, a third interconnection (113) interconnecting a second bridge point (13c) of the third unit (11) and the bridge point (14a) of the fourth unit (14), a fourth connection (141) comprising a first section (141 a) extending from the connecting point (14b) of the fourth unit (14), and a second section (141 b) extending from the first section (141 a) with a fourth passive electronic component (82) disposed thereon, wherein the fourth unit (14) is disposed under the second side of the third unit (13) by bending the fourth interconnection (113) so as to shape the PCB as a four spaced-layer structure.

11. The endoscopic device (100) of claim 9 or 10, wherein the fourth connection (141) has a width about 2mm, and the first section (141 a) has a length greater than a distance between the first unit (11) and the fourth unit (14).

12. The endoscopic device (100) of claim 9, 10 or 11, further comprising a fourth active electronic component (81) disposed on the fourth unit (14).

13. The endoscopic device (100) of claim 9, 10, 11 or 12, further comprising a first conductor (53) extending from the second section (114b) of the first connection (114), a second conductor (63) extending from the second section (121b) of the second connection (121), a third conductor (73) extending from the second section (131 b) of the third connection (131) and a fourth conductor (83) extending from the second section (141 b) of the fourth connection (141).

14. The endoscopic device (100) of any one of the preceding claims, further comprising a cover (40) covering the spaced-layer structure and comprising an transparent member (41) aligned with the image capturing assembly (20).

15. The endoscopic device (100) of claim 14, the cover (40) has a diameter less than 5mm.
